# EUROPÄISCHE PATENTSCHRIFT

(11) **EP 3 402 869 B1**
(45) Veröffentlichungstag und Bekanntmachung des Hinweises auf die Patenterteilung: **01.12.2021**
(21) Anmeldenummer: 16790877.1
(22) Anmeldetag: 21.10.2016
(51) Int. Cl.: C12M 1/00, C12M 3/00

(54) **VORRICHTUNG UND VERFAHREN ZUR AUFNAHME EINES EINWEGBEHÄLTERS**
DEVICE AND METHOD FOR ACCOMMODATING A DISPOSABLE CONTAINER
DISPOSITIF ET PROCÉDÉ DE RÉCEPTION D'UN CONTENANT JETABLE

(30) Priorität: 15.03.2016 DE 102016003136
(43) Veröffentlichungstag der Anmeldung: 21.11.2018
(73) Patentinhaber: Sartorius Stedim Biotech GmbH, 37079 Göttingen (DE)
(72) Erfinder: SCHÄFER, Jan, 34295 Edermünde (DE); SCHMID, Hannes, 34587 Felsberg (DE)
(74) Vertreter: Vigand, Philippe
(86) Internationale Anmeldenummer: PCT/EP2016/001748
(87) Internationale Veröffentlichungsnummer: WO 2017/157404

(56) Entgegenhaltungen:
- EP-A1- 2 725 092
- WO-A1-2006/032084
- WO-A1-2015/039034
- DE-A1- 4 028 871
- DE-A1-102005 006 055
- DE-A1-102010 007 559
- DE-A1-102010 018 678
- DE-U1-202007 013 406
- US-A1- 2011 310 696

## Beschreibung

Die Erfindung betrifft eine Vorrichtung und ein Verfahren zum Aufnehmen eines Einwegbehälters.

Bioreaktoren und Pallettanks dienen als Vorrichtung zur Aufnahme, Lagerung und/oder Kultivierung von biologischen Medien wie z.B. Fluiden und/oder Zellkulturen. Biologische Medien können in Einwegbehältern bereit gestellt werden, die ein Volumen von wenigen Litern bis zu mehreren hundert Litern umfassen können. Die biologischen Medien werden innerhalb eines Einwegbehälters in den Bioreaktor eingebracht, in dem sie über einen vorbestimmten Zeitraum von üblicherweise mehreren Stunden Dauer auf einer vorbestimmbaren Temperatur temperiert werden. Weiterhin können in einem solchen Bioreaktor unterschiedliche Untersuchungen an dem biologischen Medium vorgenommen werden.

Die Handhabung eines Bioreaktors kann unter Reinraumbedingungen erfolgen, so dass besonders hohe Anforderungen an die Qualitätssicherung am Bioreaktor gestellt werden.

Als Einwegbehälter können in sogenannten Single-Use-Prozessen insbesondere Einwegbeutel verwendet werden. Sowohl im Upstream als auch im Downstream erweist sich der Einsatz von Einwegbehältern als zeit- und kostensparend und prozesstechnisch sicher. Während es mittlerweile Erfahrung mit Zellkulturprozessen und einigen mikrobiellen Kulturen gibt, konnten bislang kaum Prozesse mit phototrophen Organismen wie Algen und/oder Pflanzenzellkulturen realisiert werden, insbesondere nicht für einen Produktionsmaßstab ab 50 Litern.

Das Dokument US 2011/0310696 A1 betrifft ein Fluidmixsystem mit einem Trägergehäuse, das einen Boden und aufrecht stehende Seitenwände aufweist. In einer Seitenwand ist eine Öffnung ausgebildet. Ein längliches Gestell ist benachbart zum Trägergehäuse an diesem befestigt.

Das Dokument WO 2015/039034 A1 betrifft eine Bioreaktorstützstruktur zur Aufnahme von unterschiedlichen Bioreaktoren. Die Bioreaktorstützstruktur weist zwei oder mehr Rührwerkmotoren mit Kontrollsystemen oder einen positionseinstellbaren Rührwerkmotor, einen abnehmbaren Abstandshalter und/oder Deckel, mehrere Konfigurationen für Ports und Proben und mehrere Ablassfiltererhitzer auf.

Das Dokument DE 10 2010 018 678 A1 betrifft eine Bioreaktoranordnung mit einem Bioreaktor und einem Beleuchtungskörper zur Beleuchtung eines in einem Reaktorinnenraum angeordneten Mediums. Dabei weist der Beleuchtungskörper eine Abstrahlfläche auf, über die elektromagnetische Strahlen wie Licht abgestrahlt werden, die über mindestens eine Stirnfläche in den Reaktorinnenraum eingespiegelt werden. Der Beleuchtungskörper bildet mit seiner Abstrahlfläche eine Auflage- oder Stützfläche für den Bioreaktor. Die Bioreaktoranordnung ist als eine Schüttelvorrichtung mit einer in Schwingungen versetzbaren Reaktoraufnahme für einen als Behälter mit einer transparenten Wandung ausgebildeten Bioreaktor ausgebildet, wobei die Reaktoraufnahme einen flächigen Beleuchtungskörper aufweist, dessen Abstrahlfläche für den aufzunehmenden Bioreaktor eine Auflage- oder Stützfläche bildet.

Das Dokument EP 2 725 092 A1 betrifft eine Vorrichtung für die Kultivierung phototropher Mikroorganismen in einem Photobioreaktor. Dabei wird ein Lichtelement zentral in einen zylindrischen Tank des Photobioreaktors gehängt.

Das Dokument DE 20 2007 013 406 U1 betrifft eine Beleuchtungseinrichtung für Bioreaktoren. Eine erste Ausführungsform der Beleuchtungseinrichtung ist dafür konfiguriert, von außen an einem transparenten zylindrischen Bioreaktor angeordnet zu werden, so dass sie den Reaktorinnenraum durch die transparenten Reaktorwände hindurch beleuchtet. Eine zweite Ausführungsform der Beleuchtungseinrichtung ist dafür konfiguriert, ins Innere eines zylindrischen Bioreaktors hinein gehängt zu werden. Dabei strahlt die Beleuchtungsvorrichtung in alle Richtungen Licht ab.

Der Erfindung liegt die Aufgabe zugrunde, eine Möglichkeit der eingangs genannten Art zum Aufnehmen eines Einwegbehälters mit phototrophen Organismen bereitzustellen, die insbesondere für einen Produktionsmaßstab ab 50 Litern geeignet ist.

Diese Aufgabe wird durch die Gegenstände der unabhängigen Ansprüche gelöst. Bevorzugte Ausführungsbeispiele sind die Gegenstände der abhängigen Ansprüche.

Ein erster Aspekt betrifft eine Vorrichtung zur Aufnahme eines Einwegbehälters mit einem Aufnahmebehälter mit zumindest einer Aufnahmebehälterwand, die einen Behälterinnenraum des Aufnahmebehälters zum Aufnehmen des Einwegbehälters begrenzt. Die Vorrichtung weist weiterhin eine Temperiereinheit zum Temperieren der Aufnahmebehälterwand auf. An einer dem Behälterinnenraum zugewandten Innenseite der von der Temperiereinheit temperierten Aufnahmebehälterwand ist eine Beleuchtungsvorrichtung angeordnet, die dazu ausgebildet ist, Licht in den Behälterinnenraum des Aufnahmebehälters abzustrahlen.

Die Vorrichtung kann als ein Bioreaktor und/oder Pallettank ausgebildet sein, der zur Aufnahme von Einwegbehältern mit einem Volumen von ca. 1 Liter bis ca. 2000 Litern ausgebildet sein kann, bevorzugt mit einem Volumen von ca. 200 Litern bis ca. 1000 Litern. Die Vorrichtung kann als ein System mit mehreren Komponenten ausgebildet sein, insbesondere mit oder ohne einem Einwegbehälter als zugehörige Komponente. Die Vorrichtung kann insbesondere zum Aufnehmen von Einwegbehältern ausgebildet sein, in denen sich ein biologisches Medium wie ein Fluid befindet, das über einen vorbestimmten Zeitraum im System gelagert, temperiert, kultiviert und/oder anders untersucht werden soll. Das biologische Medium kann als eine Zellkultur ausgebildet sein und/oder phototrophe Organismen aufweisen.

Der Einwegbehälter kann als ein Einwegbeutel mit flexiblen Beutelwänden ausgebildet sein, insbesondere mit transparenten Kunststoffwänden. Der Einwegbehälter kann in seinem Inneren Rührkomponenten aufweisen, die an eine Rührvorrichtung der Vorrichtung gekoppelt werden können, um so ein Vermischen des Inhalts des Einwegbehälters zu ermöglichen.

Der Aufnahmebehälter der Vorrichtung stellt den Behälterinnenraum bereit, der zum Aufnehmen des Einwegbehälters ausgebildet ist. Dabei kann der Behälterinnenraum zum Aufnehmen eines vorbestimmbaren Typs eines Einwegbehälters ausgebildet sein, also zum Beispiel eines Einwegbehälters eines vorbestimmbaren Herstellers und/oder mit einem vorbestimmbaren Füllvolumen. Die Aufnahmebehälterwand bzw. -wände definieren dabei den Behälterinnenraum. Dabei müssen die Aufnahmebehälterwande den Behälterinnenraum nicht vollständig umgeben und/oder umgrenzen. So kann der Aufnahmebehälter zum Beispiel eine Rühröffnung aufweisen, durch die eine Rührvorrichtung an den im Behälterinnenraum angeordneten Einwegbehälter anschließbar ist. Eine solche Rührvorrichtung ist bevorzugt am oberen Ende des Aufnahmebehälters ausgebildet. Der Aufnahmebehälter kann somit insbesondere deckellos und/oder oben offen ausgebildet sein.

Die Begriffe "oben", "unten", "seitlich", "vertikal", "horizontal", "Höhe", "lateral" etc. beziehen sich im Rahmen dieser Erfindung auf das Bezugssystem der Erde, in dem das System bzw. die Vorrichtung in einer Betriebsstellung angeordnet ist.

Der Einwegbehälter wird bevorzugt so in den Aufnahmebehälter eingebracht, dass er auf einem Boden des Aufnahmebehälters aufliegt und in direktem, physikalischen Kontakt mit dem Aufnahmebehälterwänden des Aufnahmebehälters steht, insbesondere in Kontakt mit dem Boden des Aufnahmebehälters und/oder den an den Boden angrenzenden Aufnahmebehälterwänden.

Die zumindest eine Aufnahmebehälterwand ist mittels der Temperiereinheit temperierbar. Die Temperiereinheit kann die Aufnahmebehälterwand auf eine vorbestimmbare, insbesondere auf eine einstellbare Temperatur temperieren. Die Temperiereinheit kann insbesondere zum Kühlen der Aufnahmebehälterwand ausgelegt sein. Dabei kann die Temperiereinheit entweder unmittelbar an einer dem Behälterinnenraum abgewandten Außenseite der Aufnahmebehälterwand angeordnet sein, oder zumindest in direktem Wärmeaustausch mit der Aufnahmebehälterwand stehen. Die Aufnahmebehälterwand kann dabei einen hohen Temperaturleitwert aufweisen, kann also z.B. metallisch ausgebildet sein.

Der Aufnahmebehälter kann zumindest eine öffenbare und schließbare Tür aufweisen, durch die der Einwegbehälter in den Behälterinnenraum eingebracht werden kann.

Im Behälterinnenraum ist die Beleuchtungsvorrichtung angeordnet, die in einer Betriebsposition der Vorrichtung zwischen der Aufnahmebehälterwand und dem Einwegbehälter angeordnet ist. In der Betriebsposition der Vorrichtung ist ein Einwegbehälter in die Vorrichtung eingebracht. Der Einwegbehälter muss kein Bestandteil der Vorrichtung sein. Der Einwegbehälter kann jedoch als ein Bestandteil der Vorrichtung ausgebildet sein.

Die Beleuchtungsvorrichtung kann so im Behälterinnenraum angeordnet sein, dass sie im Wärmeaustausch mit der temperierten Aufnahmebehälterwand steht. Dabei wird mittels der Temperiereinheit insbesondere die Beleuchtungsvorrichtung temperiert, z.B. gekühlt. Dies ermöglicht eine Wärmeabfuhr einer Beleuchtungswärme, die bei Betrieb der Beleuchtungsvorrichtung entstehen kann. Hierbei benötigt die Beleuchtungsvorrichtung keine eigene Kühlung, sondern kann mittels der Temperiereinheit der Vorrichtung temperiert bzw. gekühlt werden.

Die Vorrichtung ist insbesondere zur Aufnahme von Einwegbehältern mit phototrophen Organismen oder Zellkulturen geeignet. Mittels des von der Beleuchtungsvorrichtung abgestrahlten Lichts kann das im Einwegbehälter befindliche Medium bestrahlt werden. Hierbei sind die Wände des Einwegbehälters bevorzugt transparent für zumindest die Lichtwellenlänge(n) ausgebildet, in der die Beleuchtungsvorrichtung Licht in den Behälterinnenraum abstrahlt.

Im Rahmen dieser Erfindung bedeutet der Begriff "Licht" nicht notwendigerweise sichtbares Licht, sondern kann elektromagnetische Strahlung einer Wellenlänge von etwa 50 nm bis etwa 50 µm betreffen. Der Begriff schließt somit insbesondere ultraviolettes und infrarotes Licht mit ein. Die Wellenlänge bzw. der Wellenlängenbereich des von der Beleuchtungsvorrichtung abgestrahlten Lichts kann auf bestimmte Bedürfnisse des Mediums im Einwegbehälter abgestimmt sein. Die Beleuchtungsvorrichtung kann ein oder mehrere Lichtquellen aufweisen, die so ausgerichtet sind, dass sie Licht in Richtung von der Aufnahmebehälterwand weg zum Behälterinnenraum abstrahlen, insbesondere in einer Richtung von der Aufnahmebehälterwand weg hin zu dem Raumgebiet, in dem in der Betriebsposition der Einwegbeutel angeordnet ist, z.B. hin zu einem Mittelpunkt und/oder einer Mittelachse des Behälterinnenraums.

Die Beleuchtungsvorrichtung kann in der Betriebsposition sowohl unmittelbar an die Aufnahmebehälterwand angrenzen, als auch unmittelbar an den Einwegbehälter. Durch diese Nähe zwischen Einwegbehälter und Beleuchtungsquelle wird eine besonders effiziente Lichteinstrahlung in das Innere des Einwegbehälters mit stark reduzierten Verlusten ermöglicht. Zugleich kann über das Temperieren mit der Temperiereinheit eine negative, zu starke Hitzeentwicklung der Beleuchtungsvorrichtung vermieden werden, die ansonsten sowohl zu einer Schädigung der Zellkulturen als auch zu einer Beschädigung einer aus Kunststoff ausgebildeten Wand des Einwegbehälters führen könnte.

Damit ist die Vorrichtung besonders geeignet zur Aufnahme von Einwegbehältern, die ein Kulturvolumen für eine mikrobielle Kultur, eine Zellkultur, eine Kultur von Zell- und/oder Gewebsverbänden von pflanzlichen und/oder tierischen Zellen und/oder Hybridformen aufweisen.

Die Vorrichtung kann weiterhin eine Steuerung aufweisen, die zur Steuerung der Lichtabgabe durch die Beleuchtungsvorrichtung dient. Die Beleuchtungsvorrichtung kann z.B. modulierte und/oder nicht modulierte elektromagnetische Strahlung abstrahlen, oder auch kohärente elektromagnetische Strahlung wie z.B. Laserlicht. Der Beleuchtungsvorrichtung kann so angesteuert werden, dass sie während ansteuerbaren Zeiträumen eine ansteuerbare Lichtmenge abstrahlt. Dabei kann die Beleuchtungsvorrichtung insbesondere auch abgeschaltet werden.

Gemäß einer Ausführungsform weist die Beleuchtungsvorrichtung zumindest eine Lichtquelle auf, die an einer Position der Innenseite der Aufnahmebehälterwand angeordnet ist, die unmittelbar an einer dem Behälterinnenraum abgewandten Außenseite der Aufnahmebehälterwand von der Temperiereinheit temperiert wird. Hierbei können insbesondere alle Lichtquellen der Beleuchtungsvorrichtung an Positionen der Innenseite der Aufnahmebehälterwand angeordnet sein, die an der Außenseite temperiert sind. Da bei dieser Ausführungsform eine an der oder den Lichtquellen entwickelte Wärme sehr effizient abgeführt werden kann, ist diese Ausführungsform besonders effizient. So ist bei dieser Ausführungsform auf einer Seite der Aufnahmebehälterwand die Lichtquelle der Beleuchtungsvorrichtung angeordnet, während an der gegenüberliegenden Seite der Aufnahmebehälterwand die Temperiereinheit angeordnet ist. Hierbei kann die Temperierung durch die Aufnahmebehälterwand hindurch auf die Lichtquelle einwirken, die entweder in direkten Berührkontakt mit der Aufnahmebehälterwand angeordnet ist oder lediglich durch einen Träger von der Aufnahmebehälterwand beabstandet ist. Als Träger kann ein Substrat dienen, insbesondere ein zumindest stellenweise wärmeleitfähiges Substrat. Die Temperierung auf der Außenseite der Aufnahmebehälterwand kann z.B. mittels eines Temperiermediums erfolgen, das die Aufnahmebehälterwand an der Außenseite temperiert.

Gemäß einer Ausführungsform weist die Beleuchtungsvorrichtung eine Mehrzahl von Lichtquellen auf, die über zumindest 50% der Fläche der Innenseite aller lateralen Aufnahmebehälterwände verteilt angeordnet sind. Durch den Einsatz einer Mehrzahl von Lichtquellen wird die Lichtleistung und/oder die Anzahl von Photonen erhöht, die in den Behälterinnenraum abgestrahlt werden können. Die Lichtquellen können so angeordnet sein, dass an möglichst vielen Aufnahmebehälterwänden Lichtquellen angeordnet sind. Ein Ziel kann hierbei eine weitgehend gleichmäßige Beleuchtung über die Außenflächen des Behälters sein. Bei dieser Ausführungsform sind zumindest an 50% der lateralen Aufnahmebehälterwände Lichtquellen angeordnet. Die lateralen Aufnahmebehälterwände sind hierbei diejenigen Wände des Aufnahmebehälters, die den Behälterinnenraum in einer lateralen Richtung begrenzen. Dies sind z.B. bei einem im Wesentlichen zylinderförmigen, aufrechtstehenden Aufnahmebehälter alle Wände des Zylindermantels ohne den Zylinderboden und ohne den Zylinderdeckel. Bevorzugt können hierbei die Lichtquellen an im Wesentlichen allen lateralen Aufnahmebehälterwänden angeordnet sein. Hierbei bedeutet "im Wesentlichen", dass diejenigen Flächen der lateralen Aufnahmebehälterwände ausgespart bleiben können, an denen eine Tür und/oder ein Sichtfenster angeordnet ist. Die Lichtquellen können dabei im Wesentlichen von unten bis oben an den Aufnahmebehälterwänden angeordnet sein, insbesondere von einem Fuß des Aufnahmebehälters bis zu einer vorgesehenen Füllhöhe.

Gemäß einer Ausführungsform weist die Beleuchtungsvorrichtung eine Mehrzahl ansteuerbarer LEDs als Lichtquellen auf. Dadurch kann sowohl modulierte als auch unmodulierte Strahlung abgegeben werden. LEDs eignen sich als besonders effiziente Lichtquellen, da LEDs in Relation zur abgegebenen Lichtmenge relativ wenig Wärme entwickeln, wodurch die Wärmeentwicklung reduziert werden kann und zugleich die abgestrahlte Lichtleistung erhöht werden kann. Weiterhin können LEDs mit geringer Bauhöhe ausgebildet sein, so dass diese Lichtquellen im Wesentlichen flächig an die Innenseite der Aufnahmebehälterwand angeordnet werden können, ohne das Volumen des Behälterinnenraums zu stark zu reduzieren und ohne im Inneren des Aufnahmebehälters signifikante Vorsprünge auszubilden, die ein Einbringen des Einwegbehälters behindern würden.

Gemäß einer Ausführungsform strahlt die Beleuchtungsvorrichtung zumindest 90% der abgestrahlten Lichtleistung mit einer vorbestimmten Wellenlänge oder einem vorbestimmten Wellenlängenspektrum zwischen 50 nm und 50 µm ab. Die Beleuchtungsvorrichtung kann somit dazu ausgebildet sein, elektromagnetische Strahlung mit der vorbestimmten Wellenlänge oder einem vorbestimmten Wellenlängenspektrum abzustrahlen. Hierbei kann es sich insbesondere um Laserlicht und/oder Licht von LEDs handeln. Die vorbestimmte Wellenlänge oder das vorbestimmte Wellenlängenspektrum kann auf den Inhalt des Einwegbehälters abgestimmt sein, insbesondere auf Wellenlängen, welche den Stoffwechsel der darin befindlichen Zellen beeinflussen. Hierbei kann die Beleuchtungsvorrichtung z.B. dazu ausgebildet sein, zumindest 90% der abgestrahlten Lichtleistung bei der vorbestimmten Wellenlänge oderin dem vorbestimmten Wellenlängenspektrum abzustrahlen.

Gemäß einer Ausführungsform ist die Beleuchtungsvorrichtung dazu ausgebildet, Licht mittels auswechselbaren Lichtquellen, die unterschiedliche Abstrahlspektren aufweisen, abzustrahlen. So kann die Vorrichtung zunächst für ein Kulturmedium verwendet werden, das elektromagnetische Strahlung einer ersten Wellenlänge und/oder eines ersten Wellenlängenspektrums benötigt, die von einem ersten Satz Lichtquellen der Beleuchtungsvorrichtung abgestrahlt wird. Anschließend können die Lichtquellen ausgewechselt werden gegen Lichtquellen, die vornehmlich Licht einer zweiten Wellenlänge und/oder eines zweiten Wellenlängenspektrums abstrahlen, welche(s) für eine andere Art von Zellen oder Stoffwechsel-Beeinflussung geeignet sein kann. Die auswechselbare Ausbildung der Lichtquellen dient einerseits dazu, gegebenenfalls beschädigte einzelne Lichtquellen auswechseln zu können, als auch zur Änderung des Abstrahlungsspektrums der Beleuchtungsvorrichtung, z.B. durch Auswechslung aller Lichtquellen gegen unterschiedlich abstrahlende Lichtquellen. In dieser Ausführungsform kann die Beleuchtungsvorrichtung insbesondere eine Halterung aufweisen, die im Inneren des Aufnahmebehälters angeordnet und/oder befestigt ist, und kann zudem zumindest einen auswechselbaren Träger aufweisen, auf dem die Lichtquellen angeordnet sind. Diese Träger können z.B. streifenförmig ausgebildet sein, wobei auf jedem Streifen mehrere Lichtquellen angeordnet sein können. Die Träger können zusammen mit den darauf z.B. als eine Untergruppe angeordneten Lichtquellen aus der Halterung der Beleuchtungsvorrichtung ausgebaut und ausgewechselt werden. Die Träger der Lichtquellen können insbesondere einzeln ausgetauscht und/oder einzeln an der Halterung befestigt werden.

Gemäß einer Ausführungsform ist eine Außenform einer dem Behälterinnenraum abgewandten Außenseite der Beleuchtungsvorrichtung an eine Innenform der Innenseite der Aufnahmebehälterwand angepasst. Hierbei ist somit die Außenform der Beleuchtungsvorrichtung als komplementäres Gegenstück der Innenform der Aufnahmebehälterwand ausgebildet. Als solches Gegenstück schmiegt sich die Außenwand der Beleuchtungsvorrichtung an die Innenwand der Aufnahmebehälterwand. Dadurch wird die Wärmeleitung zwischen der Beleuchtungsvorrichtung und der Aufnahmebehälterwand verbessert. Hierbei kann zwischen der Aufnahmebehälterwand und der Beleuchtungsvorrichtung eine weitere Schicht angeordnet sein, z.B. eine Wärmeleitfolie und/oder ein Klebstoff.

In einer Weiterbildung dieser Ausführungsform ist die Außenform der Beleuchtungsvorrichtung konvex ausgebildet, während die Innenform der Aufnahmebehälterwand konkav ausgebildet ist. Beispielsweise kann der Aufnahmebehälter im Wesentlichen in Form eines aufrecht stehenden Zylinders ausgebildet sein, der einen im Wesentlichen kreisförmigen oder ovalen Querschnitt aufweist. Damit ist die Innenseite des Aufnahmebehälters konkav. Die Außenseite der Beleuchtungsvorrichtung ist hierbei an diese konkave Form angepasst, indem sie selbst konvex ausgebildet ist in Richtung zur Innenseite der Außenbehälterwand hin. Auch hierbei wird die Wärmeleitung zwischen der Beleuchtungsvorrichtung und der Aufnahmebehälterwand erhöht.

Gemäß einer Ausführungsform ist die Beleuchtungsvorrichtung dazu ausgebildet, Licht mit einer Lichtleistung von bis zu etwa 3000 µMol/m²s in den Behälterinnenraum abzustrahlen. Bei einer solch hohen Bestrahlungsleistung kann eine starke Hitzeentwicklung auftreten. Diese Hitzeentwicklung wird durch die Temperiereinheit kompensiert. Durch die hohe Lichtleistung kann eine besonders effiziente Kultivierung von biologischen Medien erfolgen.

Gemäß einer Ausführungsform weist die Beleuchtungsvorrichtung eine Mehrzahl auf einem Streifen angeordneter Lichtquellen auf, wobei der Streifen im Wesentlichen vertikal entlang der Aufnahmebehälterwand angeordnet ist. Dadurch wird ermöglicht, eine Mehrzahl von Lichtquellen zugleich an der Aufnahmebehälterwand anzuordnen. Dies ist insbesondere bei runden und/oder gebogenen Aufnahmebehälterwänden vorteilhaft, auf denen vertikale Streifen als Träger der Lichtquellen angeordnet werden. Die Streifen können entweder als Träger mehrerer vertikal voneinander beabstandeter Lichtquellen ausgebildet sein, oder als jeweils eine längliche Lichtquelle tragend, z.B. eine längliche und/oder stabförmige Lichtquelle. Entlang der Krümmung der Aufnahmebehälterwand können eine Mehrzahl solcher Streifen in dem Aufnahmebehälter angeordnet werden. Die Streifen können an einer Position im Aufnahmebehälter angeordnet sein, an der die Aufnahmebehälterwand lediglich eindimensional gekrümmt ausgebildet ist, also lediglich ein Krümmung in einer Ausbreitungsrichtung aufweist. In eine Richtung senkrecht zur Krümmungsrichtung kann die Aufnahmebehälterwand ungekrümmt ausgebildet sein. Die Streifen können so im Aufnahmebehälter angeordnet sein, dass sie sich diese ungekrümmte Ausbreitungsrichtung entlang der Aufnahmebehälterwand erstrecken, in der die Aufnahmebehälterwand im Wesentlichen gerade ausgebildet ist. Die Streifen können als Träger und/oder Substrat für die Lichtquellen ausgebildet sein. Weiterhin können die Streifen als Wärmebrücken zwischen den Lichtquellen und der Aufnahmebehälterwand ausgebildet sein und/oder solche Wärmebrücken aufweisen. Zusätzlich kann zwischen der Aufnahmebehälterwand und den Streifen eine Wärmeleitfolie und/oder eine Klebeschicht angeordnet sein.

Gemäß einer Ausführungsform steht in einer Betriebsposition der Vorrichtung der Einwegbehälter in Berührkontakt mit der Beleuchtungsvorrichtung. Dies bedeutet, dass die Beleuchtungsvorrichtung so angeordnet ist, dass in der Betriebsposition der Vorrichtung, in der der Einwegbehälter in den Aufnahmebehälter eingebracht ist, der Einwegbehälter unmittelbar auf der Beleuchtungsvorrichtung aufliegt. Dies ermöglicht eine besonders effiziente oder verlustarme Beleuchtung des Inhalts des Einwegbehälters.

Gemäß einer Ausführungsform ist über Lichtquellen der Beleuchtungsvorrichtung eine insbesondere transparente Harzschicht und/oder Lackschicht mit einer Dicke von bis zu etwa 1 mm angeordnet. Mit der Harzschicht und/oder Lackschicht können scharfe Kanten der Beleuchtungsquellen geglättet werden, die den Einwegbehälter beschädigen könnten. Weiterhin können mit der Harzschicht und/oder Lackschicht Vorsprünge reduziert werden, um die durch die Beleuchtungsvorrichtung verengten Aufnahmebehälterwände zu glätten. Als Harz kann ein Epoxidharz verwendet werden. Die Harzschicht und/oder Lackschicht ist insbesondere transparent für diejenige oder diejenigen Wellenlänge(n) ausgebildet, die von der Beleuchtungsvorrichtung abgestrahlt werden. Bevorzugt weist die Harzschicht und/oder Lackschicht auf den Lichtquellen eine maximale Dicke von etwa einem halben Millimeter auf.

Gemäß einer Ausführungsform weist die Beleuchtungsvorrichtung zumindest eine Wärmebrücke zwischen Lichtquellen der Beleuchtungsvorrichtung und der temperierten Aufnahmebehälterwand auf. Solche Wärmebrücken verbessern die Wärmeabfuhr von den Lichtquellen über die Aufnahmebehälterwand.

Gemäß einer Ausführungsform ist die Temperiereinheit dazu ausgebildet, eine Wärmeabführleistung von bis zu mindestens 8 kW zu erbringen, bevorzugt ist zumindest 10 kW, besonders bevorzugt bis zu mindestens 20 kW. Eine solch starke Wärmeabfuhrleistung der Temperiereinheit kann eine nahezu ebenso große Wärmeentwicklung bei der Beleuchtung des Inhalts der Einwegbehälter erlauben. Somit wird von dieser Ausführungsform eine besonders helle, starke und hochleistungsfähige Beleuchtung eines biologischen Mediums ermöglicht.

Gemäß einer Ausführungsform ist die Aufnahmebehälterwand zumindest teilweise als eine Temperierhohlwand ausgebildet. Hierbei ist die Temperiereinheit dazu ausgebildet, mit einem in der Temperierhohlwand angeordneten Temperiermedium den Behälterinnenraum zu temperieren.

Die Temperierhohlwand ist in den Aufnahmebehälterwänden des Aufnahmebehälters ausgebildet kann als Teil eines geschlossenen Temperierkreislaufs ausgebildet sein. Die Temperierhohlwand kann als Teil der Aufnahmebehälterwände ausgebildet sein. Die Temperierhohlwand kann als eine Doppelwand mit einer Temperierinnen- und einer Temperieraußenwand ausgebildet sein. Dabei kann die Temperierinnenwand dem Behälterinnenraum des Aufnahmebehälters zugewandt sein, und die Temperieraußenwand dem Behälterinnenraum abgewandt sein.

In einem Hohlraum zwischen der Temperierinnenwand und der Temperieraußenwand ist das Temperiermedium ausgebildet und angeordnet. Die Temperiereinheit temperiert den Behälterinnenraum, insbesondere den Inhalt des Einwegbehälters und/oder die Beleuchtungsvorrichtung mittels des Temperiermediums. Dazu kann eine Steuerung vorgesehen sein, mittels der die Temperatur und/oder der Druck des Temperiermediums in der Temperierhohlwand steuerbar und/oder einstellbar ist. Die Steuerung kann als Teil der Temperiereinheit ausgebildet sein. Die Temperiereinheit kann somit die Steuerung und/oder das in der Temperierhohlwand angeordnete Temperiermedium umfassen.

Das Innere der Temperierhohlwand kann als freier Hohlraum oder mit zumindest einem Strömungsführungselement zur Strömungsführung des Temperiermediums durch die Temperierhohlwand ausgebildet sein.

Die beiden Wände der Temperierhohlwand, also eine Temperierinnenwand und eine Temperieraußenwand, können einen Abstand von einigen Millimetern zueinander aufweisen und z.B. zwischen 5 mm und 40 mm voneinander beabstandet sein.

Die Innenseite der Temperierhohlwand, also die dem Behälterinnenraum zugewandte Temperierinnenwand, kann dabei direkt an den Behälterinnenraum und/oder die Beleuchtungsvorrichtung angrenzend ausgebildet sein. Mit anderen Worten kann zwischen dem Behälterinnenraum und der Temperierhohlwand kein weiteres Element der Vorrichtung mehr ausgebildet sein. Mit anderen Worten kann die Temperierhohlwand den Behälterinnenraum und/oder die Beleuchtungsvorrichtung somit zumindest teilweise unmittelbar umgeben und/oder begrenzen.

Dabei bedeutet "zumindest teilweise umgeben", dass die Temperierhohlwand den Behälterinnenraum an seiner Außenfläche zumindest zu 40% umgibt, bevorzugt zumindest zu 60% umgibt, besonders bevorzugt zumindest zu 70% umgibt, besonders bevorzugt zumindest zu 80% umgibt. Insbesondere kann dies bedeuten, dass der Behälterinnenraum vollständig bis auf einen oberen Deckelbereich und ggf. Sichtfenster und/oder eine Türöffnung von der Temperierhohlwand umgeben und/oder umgrenzt und/oder temperiert wird.

Die Temperierhohlwand kann als ein Druckgerät nach PED-Richtlinie ("Pressure Equipment Directive" für den europäischen Wirtschaftsraum) und/oder nach ASME-Richtlinie ("American Society of Mechanical Engineers" für den amerikanischen Wirtschaftsraum) ausgebildet sein, insbesondere als ein Druckgerät mit etwa 6 barg.

Allgemein kann das Temperiermedium als ein Fluid wie zum Beispiel eine Flüssigkeit oder ein Gas ausgebildet sein, das sich in einem abgeschlossenen Temperierkreislauf befindet. Zumindest einen Teil dieses Temperierkreislaufs stellt die Temperierhohlwand des Aufnahmebehälters dar.

Gemäß einer Ausführungsform ist der Aufnahmebehälter dazu ausgebildet, Einwegbehälter mit einem Volumen von zumindest etwa 50 Litern aufzunehmen, bevorzugt zumindest 1000 Litern, besonders bevorzugt zumindest etwa 2000 Litern. Bei vorbekannten Vorrichtungen war es bislang nicht möglich, Einwegbehälter mit solch großen Volumina effizient zu beleuchten und zu kultivieren. Erst die besonders effiziente Beleuchtung und Kühlung durch die erfindungsgemäße Vorrichtung ermöglicht die hierfür benötigte Lichtbeaufschlagung. Dabei ist zu beachten, dass das von der Beleuchtungsvorrichtung emittierte Licht unter Umständen zwar nicht vollständig bis zur Mitte des Mediums in dem Einwegbehälter vordringt, aber durch eine Vermischung des Mediums mittels einer Rührvorrichtung trotzdem eine ausreichend gleichmäßige Beleuchtung des Mediums bereitgestellt werden kann.

Die Vorrichtung kann die GMP-Richtlinien erfüllen. Die GMP Richtlinien, was abgekürzt für "Good Manufacturing Practice" steht, sind Richtlinien über Anforderungen an Hygiene, Räumlichkeiten, Ausrüstung, Dokumentation und Kontrollen im pharmazeutischen Bereich. Die Vorrichtung kann so ausgebildet sein, dass sie die in den GMP Richtlinien genannten Anforderungen erfüllt, insbesondere die Anforderungen an Sterilität.

Ein zweiter Aspekt betrifft ein Verfahren zur Aufnahme eines Einwegbehälters mit den Schritten:
- Aufnehmen eines Einwegbehälters in einen Behälterinnenraum eines Aufnahmebehälters mit zumindest einer Aufnahmebehälterwand, die den Behälterinnenraum begrenzt;
- Beleuchten des im Behälterinnenraum angeordneten Einwegbehälters mittels einer Beleuchtungsvorrichtung, die an einer dem Behälterinnenraum zugewandten Innenseite der Aufnahmebehälterwand angeordnet ist; und
- Temperieren der Aufnahmebehälterwand.

Das Verfahren kann insbesondere mit der Vorrichtung gemäß dem ersten Aspekt durchgeführt werden. Deswegen beziehen sich alle im Zusammenhang mit der Vorrichtung gemachten Ausführungen auch auf das Verfahren gemäß dem zweiten Aspekt und umgekehrt.

Die Erfindung wird nachfolgend anhand von in Figuren gezeigten Ausführungsbeispielen näher beschrieben. Gleiche Bezugszeichen bezeichnen gleiche oder ähnliche Bauteile der Ausführungsformen. Einzelne Merkmale der Ausführungsformen können mit anderen Ausführungsformen kombiniert werden. Es zeigen:
- Figur 1: in einer perspektivischen Darstellung eine Vorrichtung zum Aufnehmen eines Einwegbeutels;
- Figur 2A: in einer ersten Seitenansicht eine Vorrichtung zum Aufnehmen eines Einwegbeutels;
- Figur 2B: in einer zweiten Seitenansicht eine Vorrichtung zum Aufnehmen eines Einwegbeutels;
- Figur 3: in einer perspektivischen Darstellung eine vertikale Schnittansicht durch eine Vorrichtung zum Aufnehmen eines Einwegbeutels;
- Figur 4: in einer schematischen Darstellung eine Vorrichtung zum Aufnehmen eines Einwegbeutels mit einer Beleuchtungsvorrichtung;
- Figur 5: einen schematisch dargestellten Querschnitt durch eine Beleuchtungsvorrichtung für einen Aufnahmebehälter;
- Figur 6A: einen schematisch dargestellten Querschnitt durch einen Ausschnitt eines Beleuchtungsträgers; und
- Figur 6B: einen schematisch dargestellten Querschnitt durch einen Ausschnitt eines Beleuchtungsträgers.

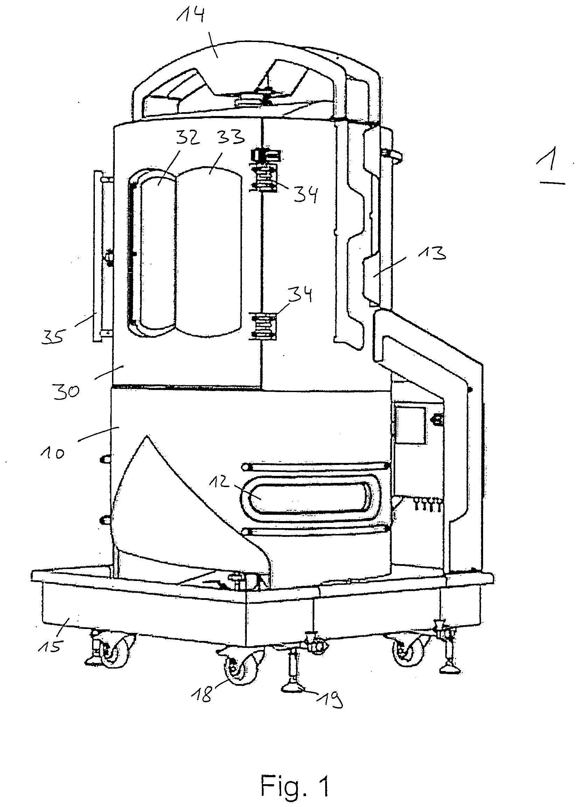

**Figur 1** zeigt in einer perspektivischen Darstellung eine Vorrichtung 1 zum Aufnehmen eines Einwegbeutels als Einwegbehälter. Die in den Figuren gezeigte Vorrichtung 1 kann als ein Bestandteil eines Systems zum Aufnehmen eines Einwegbeutels ausgebildet sein.

Die Vorrichtung 1 weist einen Aufnahmebehälter 10 auf, der im Wesentlichen die Form eines vertikal angeordneten Zylinders aufweist, d.h. dessen Zylinderachse im Wesentlichen vertikal angeordnet ist. Der Aufnahmebehälter 10 weist einen Behälterinnenraum auf, in den ein Einwegbeutel eingebracht werden kann, der zum Beispiel ein biologisches Medium beinhalten kann. Das biologische Medium im Einwegbeutel wird im Behälterinnenraum des Aufnahmebehälters 10 über einen vorbestimmbaren Zeitraum gelagert und/oder beleuchtet. Während sich der Einwegbeutel mit dem biologischen Medium im Inneren des Aufnahmebehälters 10 befindet, können sich unterschiedliche Reaktionen mit oder an dem biologischen Medium ereignen. Somit kann die Vorrichtung 1 auch als Bioreaktor ausgebildet sein.

Zur Beobachtung des biologischen Mediums sind in den Seitenwänden ein oder mehrere Sichtfenster ausgebildet, durch das bzw. die von außen durch die Aufnahmebehälterwand hindurch in den Behälterinnenraum des Aufnahmebehälters 10 hineingeblickt werden kann, um das biologische Medium zu beobachten. Die Vorrichtung 1 weist dazu im unteren Drittel zwei Bodensichtfenster 12 auf, sowie ein Türsichtfenster 32. Die Bodensichtfenster 12 sind im Wesentlichen in Form eines länglichen Ovals ausgebildet, dessen lange Ovalachse im Wesentlichen horizontal entlang der gekrümmten Zylinderaußenwand des Aufnahmebehälters 10 ausgerichtet ist. Das Türsichtfenster 32 ist im Wesentlichen in Form eines länglichen Rechtecks ausgebildet, wobei deren längere Seiten im Wesentlichen vertikal ausgerichtet sind und in der Mitte einer Einblatttür 30 in der Behälterwand des Aufnahmebehälters 10 ausgebildet sind.

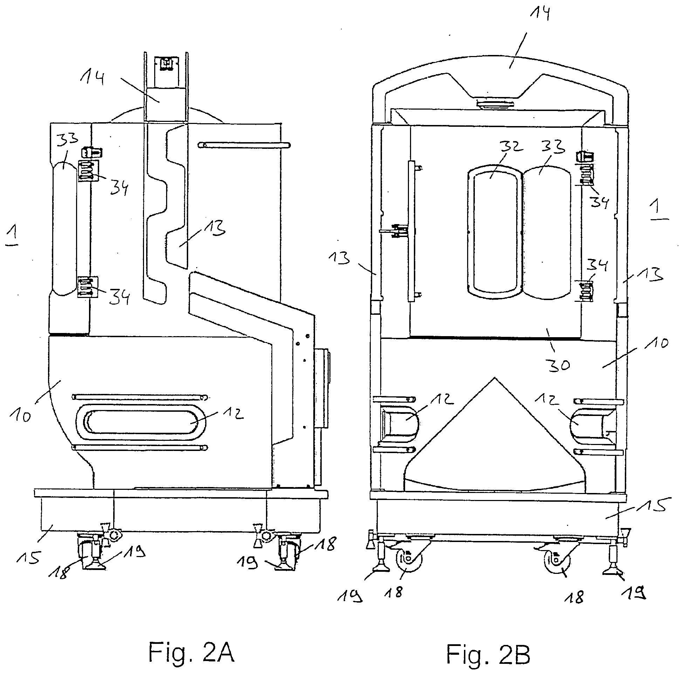

**Figuren 2A und 2B** zeigen zusammen mit Figur 1 unterschiedliche Ansichten der Vorrichtung 1. So zeigt z.B. Figur 2B eine frontale Seitenansicht auf die Einblatttür 30. Die Einblatttür 30 erstreckt sich in der Breite, also in horizontaler Richtung, in etwa über ein Zylindersegment des Aufnahmebehälters 10 von ca. 100°. In horizontaler Richtung erstreckt sich die Einblatttür 30 von zwei Türscharnieren 34 entlang des Zylindermantels bis zu einem Türgriff 35 am gegenüberliegenden Türende. Die Einblatttür 30 ist im Wesentlichen in den oberen zwei Dritteln des Aufnahmebehälters 10 ausgebildet, während das untere Drittel des Aufnahmebehälters 10 im Wesentlichen in Form einer steifen Bodenschale ausgebildet ist, die selber nicht öffenbar ausgebildet ist. Die Einblatttür 30 ist um die Türscharniere 34 drehbar und somit offenbar. Ist die Einblatttür 30 geöffnet, so ist in dem Aufnahmebehälter 10 an einer seitlichen Position eine Türöffnung ausgebildet, durch die ein Zugriff auf den Behälterinnenraum des Aufnahmebehälters 10 ermöglicht wird. Durch die Türöffnung kann zum Beispiel der Einwegbeutel von einer seitlichen Richtung her, also im Wesentlichen in einer horizontalen Bewegungsrichtung, in den Behälterinnenraum des Aufnahmebehälters 10 eingeführt werden.

Die Vorrichtung 1 ist auf Rollen 18 rollbar gelagert, auf denen die Vorrichtung rollend durch einen Raum geschoben werden kann. Zusätzlich zu den Rollen 18 kann die Vorrichtung 1 am unteren Ende Fixierfüße 19 aufweisen, die zum Fixieren und korrektem Ausrichten der Vorrichtung 1 z.B. auf unebenen Böden dienen.

Der Aufnahmebehälter 10 ist nach oben offen ausgebildet. An Stelle eines Zylinderdeckels weist der Aufnahmebehälter 10 eine Rühröffnung auf. Oberhalb des nach oben offenen Aufnahmebehälters 10 ist eine Rührvorrichtung 14 ausgebildet, über die ein Rührstab durch die Rühröffnung hindurch mit dem Einwegbeutel so verbunden werden kann, dass das Innere des Einwegbeutels durchmischt werden kann. Der Rührstab kann im Inneren des Einwegbeutels angeordnet sein und über eine Kopplung bzw. Kupplung mit der Rührvorrichtung 14 verbunden werden. Die Rührvorrichtung 14 ist zentral über dem Aufnahmebehälter 10 ausgebildet und wird von einer Trägerbrücke getragen, die auf einem oberen Rand des Aufnahmebehälters 10 an einander gegenüberliegenden Seitenwänden des Aufnahmebehälters 10 aufliegt.

Figur 2A zeigt eine Seitenansicht auf eine der beiden seitlich angeordneten Kabelführungen 13. Die zweite der beiden Kabelführungen 13 ist an der gegenüberliegenden Außenwand des Aufnahmebehälters 10 angeordnet. Die Seitenansicht der Figur 2A zeigt die Vorrichtung 1 in einer Position, die gegenüber der Seitenansicht der Figur 2B um 90° gedreht ist.

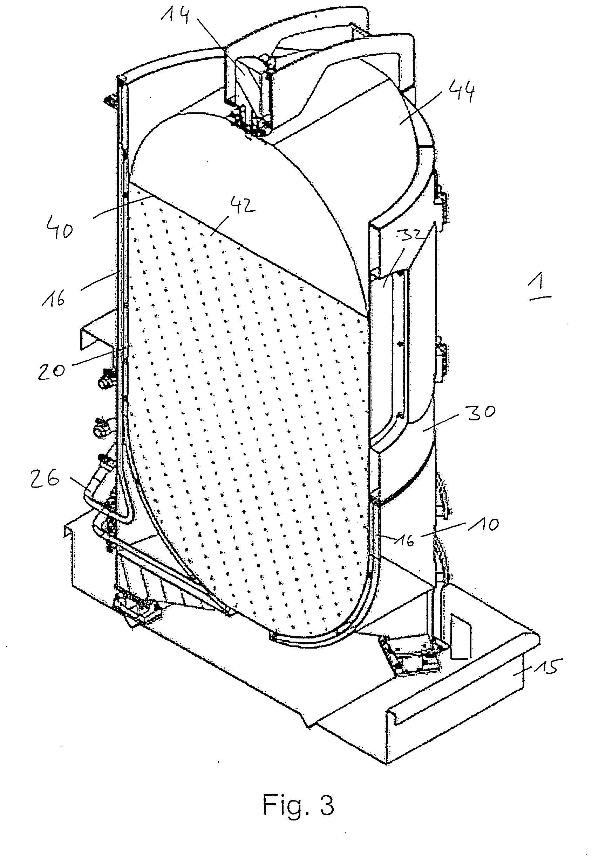

**Figur 3** zeigt in einer perspektivischen Darstellung eine Ansicht eines vertikalen Schnitts durch die Vorrichtung 1, auf eine Darstellung einer Beleuchtungsvorrichtung verzichtet wurde. In Figur 3 gezeigt ist z.B. ein Einwegbeutel 44 als Einwegbehälter, genauer ein Schnitt durch diesen Einwegbeutel 44, der im Behälterinnenraum des Aufnahmebehälters 10 angeordnet ist. Im Behälterinnenraum des Aufnahmebehälters 10 und zugleich auch im Inneren des Einwegbeutels 44 ist ein biologisches Medium 42 angeordnet, das bis auf Höhe einer vorbestimmten Füllstandhöhe 40 gefüllt ist. Das biologische Medium 42 erstreckt sich vom Boden des Aufnahmebehälters 10 bis hin zur Füllstandhöhe 40 und füllt somit das gesamte Innenvolumen des Aufnahmebehälters 10 bis hin zur Füllstandhöhe 40, abzüglich des Volumens der Wände des Einwegbeutels 44 und einer nicht gezeigten Beleuchtungsvorrichtung.

Der Einwegbeutel 44 wird durch eine Behälterwand 16 des Aufnahmebehälters 10 in Form gehalten, die sich vom abgerundeten Boden des Aufnahmebehälters 10 bis über die Füllstandhöhe 40 hinaus nach oben erstrecken kann. Zumindest entlang der oberen Hälfte, bevorzugt entlang der oberen zwei Drittel des Aufnahmebehälters 10 kann sich eine Aufnahmebehälterwand 16 im Wesentlichen in vertikaler Richtung senkrecht nach oben erstrecken.

Die Aufnahmebehälterwände 16 des Aufnahmebehälters 10 sind zumindest teilweise als eine Temperierhohlwand 20 ausgebildet, in der ein (in den Figuren nicht gezeigtes) Temperiermedium fließt. Das Temperiermedium kann auf einen Niedrigdruck kleiner als 0,5 bar geregelt werden, oder auf einen Druck bis etwa 6 barg geregelt werden. Die Temperierhohlwand 20 kann sich über den gesamten Boden bzw. die gesamte Bodenschale des Aufnahmebehälters 10 und entlang der Aufnamebehälterwände 16 vom Behälterboden nach oben bis über die Füllstandhöhe 40 hinaus bis zu einer vorbestimmbaren Temperierhöhe erstrecken. Die Temperierhöhe kann im Wesentlichen 1 cm bis 20 cm vertikal oberhalb der Füllstandhöhe 40 angeordnet sein.

Das biologische Medium 42 kann in Wärmekontakt zu der Temperierhohlwand 20 stehen, von der es lediglich über die dünne Beutelwand des Einwegbeutels 44 und zumindest teilweise eine nicht gezeigte Beleuchtungsvorrichtung getrennt ist. Über das Temperiermedium kann das biologische Medium 42 und/oder die Beleuchtungsvorrichtung auf eine vorbestimmbare Temperatur geregelt werden.

Die Vorrichtung 1 kann insbesondere dazu ausgebildet und vorgesehen sein, den Behälterinnenraum und die darin angeordnete Beleuchtungsvorrichtung auf eine vorbestimmbare Solltemperatur von etwa 0°C bis etwa 80°C, bevorzugt von etwa 20°C bis etwa 40°C zu temperieren.

Die Temperierhohlwand 20 umgibt den Behälterinnenraum des Aufnahmebehälters 10 bis über die Füllstandhöhe 40 hinaus beinahe vollständig. "Beinahe vollständig umgeben" bedeutet in den in den Figuren gezeigten Ausführungsbeispielen, dass die Temperierhohlwand 20 den Behälterinnenraum bis zur Temperierhöhe hin vollständig bis auf diejenigen Positionen umgibt, an denen die ggf. vorhandenen Bodensichtfenster 12 angeordnet sind und an der die zumindest eine Einblatttür 30 angeordnet ist. An den Positionen der Bodensichtfenster 12 kann eine Glasscheibe angeordnet sein, die Sicht auf den Behälterinnenraum und insbesondere das im Aufnahmebehälter 10 befindliche biologische Medium 42 gewährt (und gegebenenfalls eine entsprechende Verdunklung ohne Temperierung). An der Position der Einblatttür 30 befindet sich eine Aussparung in Form der Türöffnung 36 in der Temperierhohlwand 20. In einer alternativen Ausführungsform kann auch an der Innenseite der Einblatttür 30 eine Temperierhohlwand ausgebildet sein, die über an den Türscharnieren 34 angeordnete Temperierleitungen mit dem Temperiermedium versorgt wird.

Allgemein kann "beinahe vollständig umgeben" bedeuten, dass die Temperierhohlwand 20 den Behälterinnenraum bis zur Temperierhöhe hin vollständig bis auf einige wenige vorbestimmte Positionen umgibt. Diese einigen wenigen Positionen können die Positionen sein, an denen Sichtfenster in der Behälterwand des Aufnahmebehälters 10 angeordnet sind, und gegebenenfalls an der die Einblatttür 30 angeordnet ist. Im Allgemeinen begrenzt die Temperierhohlwand 20 den Behälterinnenraum nicht an dessen oberen Ende.

Die Temperierhohlwand 20 kann an ihrer Außenseite, also der dem Behälterinnenraum abgewandten Seite, von einer Isolierhohlwand umgeben sein, in der sich eine Isolierung befindet. Die Isolierhohlwand kann den Aufnahmebehälter 10 beinahe vollständig vom Boden des Aufnahmebehälters 10 bis hin zum oberen Ende der Aufnahmebehälterwand 16 (vgl. dazu auch Figur 3) umgeben. Die Isolierhohlwand isoliert sowohl den Behälterinnenraum, als auch insbesondere die Temperierhohlwand 20 nach außen hin. Durch die in der Isolierhohlwand angeordnete Isolierung wird eine gerichtete Temperierung mittels des Temperiermediums nach innen hin zum Behälterinnenraum und/oder der Beleuchtungsvorrichtung bereitgestellt, was die Energieeffizienz der Vorrichtung 1 erhöht.

Temperierleitungen können mit dem Inneren der Temperierhohlwand 20 verbunden sein. Die Vorrichtung 1 kann weiterhin Anschlüsse bzw. Isolierleitungen zum bereitstellen eines Vakuums in der Isolierhohlwand aufweisen, die in den Figuren nicht gezeigt sind.

Die Temperierhöhe kann sowohl die vorbestimmte Füllstandshöhe 40 übersteigen, als auch die Kontaktfläche des Einwegbeutels 44 mit den Aufnahmebehälterwänden 16 des Aufnahmebehälters 10.

Der Einwegbeutel 44 wird nach der Verwendung zum Beispiel über ein unterhalb der Vorrichtung 1 angeordneten Auslass entleert und kann anschließend vollständig entsorgt werden. Durch Verwendung des Einwegbeutels 44 in einem Single-Use-Prozess kann eine Reinigung der Vorrichtung 1 eingespart werden, bzw. wesentlich schneller erfolgen.

Eine Auffangwanne 15 kann als Auffangelement dienen, falls aus der Vorrichtung 1 z.B. durch ein Leck im Einwegbeutel biologisches Medium austreten sollte.

Als Isolierung in der Isolierhohlwand kann Luft, ein Vakuum, eine Dämmwolle, eine Glaswolle, eine Steinwolle, oder ein ähnliches Isoliermaterial verwendet werden.

Die Vorrichtung 1 weist im Inneren der Temperierhohlwand 20 möglichst wenig Wärmebrücken auf, also z.B. durchgehende Metallverbindungen vom Behälterinnenraum zum Außenraum, welche die Temperierung des Behälterinnenraums verlangsamen würden. Die Vorrichtung 1 kann lediglich statisch notwendige und/oder statisch zwingend erforderliche Wärmebrücken aufweisen.

Das Temperiermedium befindet sich im Inneren eines abgeschlossenen Temperiersystems, das das Innere der Temperierhohlwand 20 umfasst. Die Regelung und/oder Steuerung der Temperatur des Temperiermediums kann über eine interne, elektrisch betriebene Heizvorrichtung erfolgen sowie optional oder alternativ über einen externen Wärmetauscher. Über den externen Wärmetauscher kann sowohl eine Kühlung als auch eine Erwärmung des Temperiermediums unabhängig von der internen elektrischen Heizvorrichtung des Temperiersystems erfolgen.

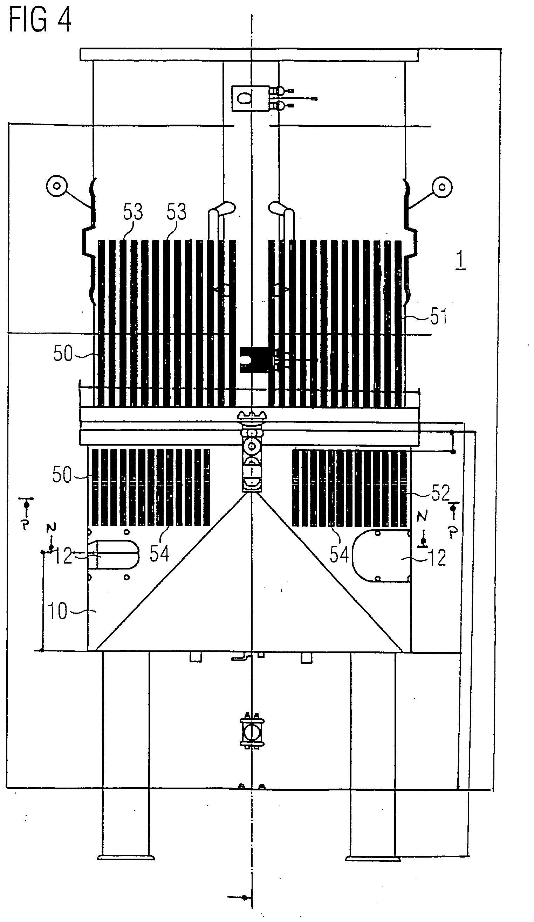

Figur 4 zeigt in einer schematischen Darstellung eine Vorrichtung 1 mit dem Aufnahmebehälter 10, in dem eine mehrteilige Beleuchtungsvorrichtung 50 angeordnet ist. Die Beleuchtungsvorrichtung 50 weist eine erste, obere Beleuchtungseinheit 51 sowie eine zweite, untere Beleuchtungseinheit 52 auf. Allgemein kann die Beleuchtungsvorrichtung 50 mehrere Beleuchtungseinheiten 51, 52 aufweisen, die z.B. wie im gezeigten Ausführungsbeispiel übereinander angeordnet sein können.

Figur 4 zeigt dabei eine schematische Darstellung der Vorrichtung, in der insbesondere nicht die Temperiereinheit und auch nicht die doppelwandige Aufnahmebehälterwand gezeigt ist, die mit Bezug auf die voranstehenden Figuren beschrieben sind. Die Vorrichtung 1 kann jedoch alle Elemente der mit Bezug auf die vorangegangenen Figuren beschriebene Vorrichtung aufweisen.

Die Beleuchtungsvorrichtung 50 ist im Inneren des Aufnahmebehälters 10 angeordnet. Dabei ist die obere Beleuchtungseinheit 51 in einem oberen Teil des Aufnahmebehälters 10 angeordnet, während die untere Beleuchtungseinheit 52 in einem unteren Teil des Aufnahmebehälters 10 angeordnet ist, z.B. im Inneren einer Bodenschale des Aufnahmebehälters 10. Die obere Beleuchtungseinheit 51 ist in dem oberen Teil des Aufnahmebehälters 10 angeordnet, an dem auch die Einblatttür 30 zum Einbringen des Einwegbehälters angeordnet ist (vgl. z.B. Figur 1). Die obere Beleuchtungseinheit 51 kann im Wesentlichen ringförmig ausgebildet sein, wobei der Ring im Inneren des Aufnahmebehälters 10 entlang des Umfangs und somit entlang der inneren Seite der Aufnahmebehälterwand 16 angeordnet ist. Hierbei fällt der Ringmittelpunkt der oberen Beleuchtungseinheit 51 im Wesentlichen mit der Zylinderachse des Aufnahmebehälters 10 zusammen.

Ähnlich dazu ist auch die untere Beleuchtungseinheit 52 im Wesentlichen ringförmig ausgebildet, und zwar entlang der Innenseite des unteren Teils des Aufnahmebehälters 10. Auch der Mittelpunkt des Rings der unteren Beleuchtungseinheit 52 liegt im Wesentlichen auf der Zylinderachse des Aufnahmebehälters 10.

Die obere Beleuchtungseinheit 51 weist eine Mehrzahl oberer Beleuchtungsträger 53 auf, die im Wesentlichen stabförmig und/oder streifenförmig ausgebildet sind. Die oberen Beleuchtungsträger 53 sind im Wesentlichen parallel zueinander ausgerichtet und als vertikal angeordnete Streifen ausgebildet. Die oberen Beleuchtungsträger 53 können jeweils eine oder mehrere Lichtquellen tragen, die Licht ins Behälterinnere des Aufnahmebehälters 10 abstrahlen.

Ähnlich dazu weist auch die untere Beleuchtungseinheit 52 eine Mehrzahl von unteren Beleuchtungsträgern 54 auf. Die unteren Beleuchtungsträger 54 sind im Wesentlichen stabförmig und/oder streifenförmig ausgebildet, die in einer im Wesentlichen vertikalen Richtung entlang der Innenseite des Aufnahmebehälters 10 angeordnet sind. Sowohl die oberen Beleuchtungsträger 53 als auch die unteren Beleuchtungsträger 54 dienen zum Tragen von Lichtquellen der Beleuchtungsvorrichtung 50. Die Streifenbreite der Beleuchtungsträger 53 und 54 in horizontale Richtung ist dabei wesentlich kleiner als die Streifenlänge in vertikaler Richtung. Durch diese Anordnung können die streifenförmigen Beleuchtungsträger 53 und 54 besonders günstig und einfach an die runde Zylinderinnenwand des Aufnahmebehälters 10 angepasst sein.

Im gezeigten Ausführungsbeispiel haben die unteren Beleuchtungsträger 54 eine geringere vertikale Ausdehnung als die oberen Beleuchtungsträger 53. Die vertikale Ausdehnung der jeweiligen Beleuchtungsträger 53 und 54 können von der im Inneren des jeweiligen Teils des Aufnahmebehälters 10 vorhandenen vertikalen Höhe abhängig sein. So ist der obere Teil des Aufnahmebehälters 10, in dem die obere Beleuchtungseinheit 51 angeordnet ist, höher als der untere Teil des Aufnahmebehälters 10, in dem die untere Beleuchtungseinheit 52 angeordnet ist. In anderen Ausführungsbeispielen kann dies umgekehrt sein, oder aber die oberen und unteren Beleuchtungsträger 53 und 54 können im Wesentlichen gleich lang ausgebildet sein.

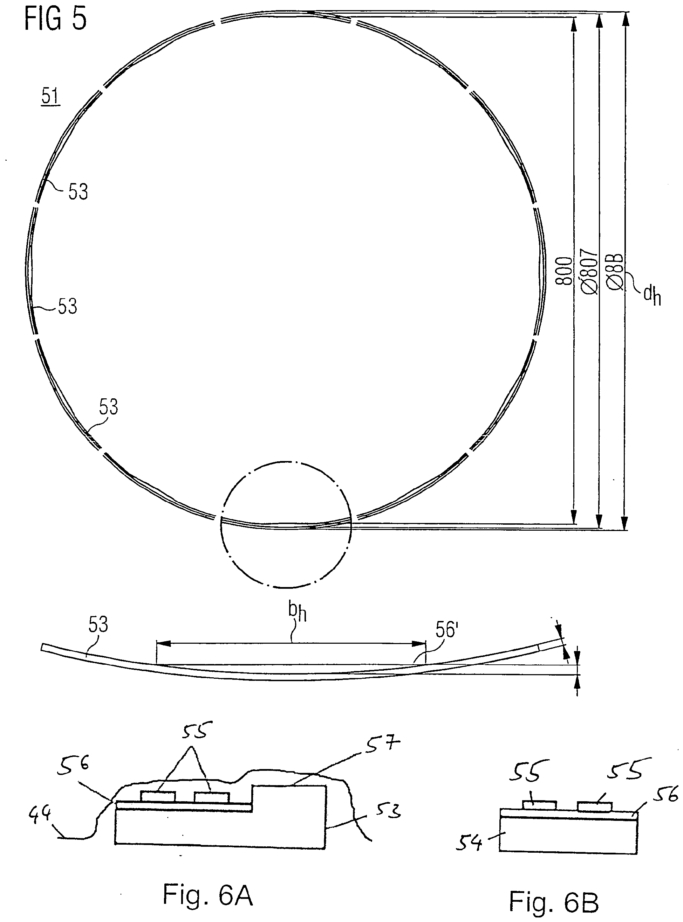

**Figur 5** zeigt einen stark schematisierten horizontalen Querschnitt durch die erste, obere Beleuchtungseinheit 51. Die obere Beleuchtungseinheit 51 ist dazu ausgebildet und vorgesehen, in den Behälterinnenraum des Aufnahmebehälters 10 eingebracht zu werden. Der Aufnahmebehälter 10 weist im horizontalen Querschnitt eine im Wesentlichen kreisförmige Aufnahmebehälterwand 16 auf. Diese Aufnahmebehälterwand 16 kann, wie voranstehend ausgeführt, als Temperierhohlwand 20 ausgebildet sein.

Wie in Figur 5 gezeigt ist die Querschnittsform der oberen Beleuchtungseinheit 51 an den Querschnitt durch den oberen Teil der Aufnahmebehälters 10 angepasst. So weisen sowohl der Aufnahmebehälter 10 als auch die obere Beleuchtungseinheit 51 im horizontalen Querschnitt eine im Wesentlichen kreisförmige Form auf, wobei die beiden Kreisdurchmesser nahezu gleich groß sind.

Die obere Beleuchtungseinheit 51 weist eine Mehrzahl gebogener, obere Beleuchtungsträger 53 auf. Jeder der oberen Beleuchtungsträger 53 weist eine nach außen konvexe Krümmung auf, die etwa der nach innen konkaven Krümmung der Innenseite der Aufnahmebehälterwand 16 entspricht. Die oberen Beleuchtungsträger 53 sind entlang der Innenseite der Aufnahmebehälterwand 16 angeordnet über ein Winkelsegment von jeweils nahezu 30°. Damit kann die Innenseite der Aufnahmebehälterwand 16 von den zwölf in Fig. 5 gezeigten Beleuchtungsträgern 53 entlang des Zylindermantels nahezu vollständig bedeckt werden. Zusammen bilden die oberen Beleuchtungsträger 53 die im Querschnitt ringförmige obere Beleuchtungseinheit 51.

Im unteren Teil der Figur 5 ist ein Ausschnitt des Querschnitts vergrößert dargestellt, der im oberen Teil der Figur 5 durch einen strichpunktierten Kreis markiert ist.

In diesem vergrößert dargestellten Querschnitt ist gezeigt, dass die einzelnen oberen Beleuchtungsträger 53 im horizontalen Querschnitt gekrümmt ausgebildet sind. Die Beleuchtungsträger 53 können Platinen 56 (vgl. hierzu auch Fig. 6A und 6B) zum ansteuern der Lichtquellen aufweisen, die üblicherweise zunächst ohne Krümmung hergestellt werden. Die Platinen 56 können auf den oberen Beleuchtungsträgern 53 ebenfalls gekrümmt angeordnet sein. Um eine Beschädigung der Platinen 56 zu vermeiden, ist dabei die Krümmung der Platinen 56 aus der ebenen Form heraus möglichst gering. Deswegen sind die oberen und unteren Beleuchtungsträger 53, 54 so ausgebildet, dass sie im horizontalen Querschnitt ein Winkelsegment von maximal etwa 45° der Innenseite der Aufnahmebehälterwand 16 bedecken, bevorzugt maximal etwa 30°, besonders bevorzugt maximal etwa 20°.

Alternativ oder zusätzlich dazu können an den oberen und unteren Beleuchtungsträgern 53, 54 auch ungekrümmte Platinen 56' angeordnet sein, die z.B. in die gekrümmten Beleuchtungsträger 53, 54 eingelegt werden, wie in Fig. 5 schematisch durch das Bezugszeichen der ungekrümmten Platinen 56' gekennzeichnet. Um ein möglichst gutes Anpassen der ungekrümmten Platinen 56' an die Beleuchtungsträger 53 und somit die Aufnahmebehälterwand 16 zu ermöglichen, ist die in Figur 5 gezeigte horizontale Ausbreitung der oberen ungekrümmten Platinen 56' wesentlich kleiner als der Durchmesser des horizontalen Querschnitts durch den Aufnahmebehälter 10 ausgebildet. So kann eine längste horizontale Ausdehnung bₕ der Platinen 56' zwischen 1% und 15% des horizontalen Innendurchmessers dₕ des Aufnahmebehälters 10 betragen, bevorzugt zwischen 1% und 10%, besonders bevorzugt zwischen 1 und 5%.

Dadurch ergibt sich die in Figur 4 gezeigte vertikal-streifenförmige Form der Beleuchtungsträger 53 und 54.

Für die in Figur 5 nicht gezeigten unteren Beleuchtungsträger 54 kann ähnliches bzw. gleiches gelten.

**Figur 6A** zeigt einen schematischen Querschnitt durch einen Ausschnitt einer Beleuchtungseinheit, z.B. der oberen Beleuchtungseinheit 51 oder der unteren Beleuchtungseinheit 52, insbesondere einen horizontalen Querschnitt durch einen der Beleuchtungsträger. Hierbei kann ein Grundkörper des z.B. oberen Beleuchtungsträgers 53 metallisch ausgebildet sein, insbesondere als eine Halterung aus einem Metall wie z.B. Aluminium mit einem hohen Wärmeleitwertkoeffizienten. Der Grundkörper kann an einer Außenseite 53a in unmittelbarem mechanischem Kontakt und somit in Wärmeaustausch mit der Innenseite der Aufnahmebehälterwand 16 stehen. Auf einer Innenseite bzw. Innenwand der z.B. oberen Beleuchtungseinheit 51 ist eine Platine 56 angeordnet. Auf der Platine 56 sind mehrere Lichtquellen 55 angeordnet, z.B. LEDs. Die Lichtquellen 55 sind so ausgerichtet, dass sie in Richtung zum Behälterinnenraum Licht abstrahlen, z.B. im Wesentlichen zur Zylinderachse des Aufnahmebehälters 10 hin, die durch den Mittelpunkt des in Figur 5 gezeigten kreisförmigen Querschnitts verläuft.

Eine Beutelwand des Einwegbehälters 44 kann im Wesentlichen unmittelbar an den Lichtquellen 55 anliegen. Da die Wand des Einwegbehälters 44 flexibel ausgebildet sein kann, insbesondere aus einem flexiblen Material, kann sich die Form des Einwegbehälters 44 im Wesentlichen dem Innenprofil der oberen Beleuchtungseinheit 51 anpassen. Auf und/oder neben den oberen Beleuchtungsträgern 53 kann die obere Beleuchtungseinheit 51 weiterhin einen oder mehrere Stege 57 aufweisen, die eine Wärmebrücke zwischen dem Einwegbeutel 44 und der Aufnahmebehälterwand 16 bilden.

**Figur 6B** zeigt einen schematischen Querschnitt durch einen Ausschnitt einer Beleuchtungseinheit mit einem Grundkörper, z.B. einen horizontalen Querschnitt durch einen der unteren Beleuchtungsträger 54 der unteren Beleuchtungseinheit 52. Der z.B. untere Beleuchtungsträger 54 kann metallisch ausgebildet sein (z.B. aus Aluminium). Der Grundkörper des unteren Beleuchtungsträgers 54 kann an einer Außenseite 54a in unmittelbarem mechanischem Kontakt und somit in Wärmeaustausch mit der Innenseite der Aufnahmebehälterwand 16 stehen. Auf einer der Außenseite 54a gegenüberliegenden Innenseite ist eine Platine 56 angeordnet, auf der Lichtquellen 55 angeordnet sind (z.B. LEDs).

Allgemein kann sowohl die obere Beleuchtungseinheit 51 als auch die untere Beleuchtungseinheit 52 einen oder mehrere der Beleuchtungsträger 53 und/oder 54 aufweisen, sowie einen oder mehrere der Stege 57. In Figur 6B ist eine Darstellung der Wand des Einwegbeutels 44 weggelassen. Hierbei sind die Figuren 6A und 6B als schematisch dargestellte Beispiele angegeben, die sich nicht unbedingt auf Ausschnitte der oberen Beleuchtungseinheit 51 oder der unteren Beleuchtungseinheit 52 beziehen müssen.

Beide Beleuchtungseinheiten 51, 52 können im Wesentlichen ringförmig entlang des Innenumfangs des Aufnahmebehälters 10 angeordnet sein.

Die Platinen 56 mit den Lichtquellen 55 können austauschbar an der oberen und/oder unteren Beleuchtungseinheit 51 bzw. 52 angeordnet sein, um ein Abstrahlspektrum der Lichtquellen 55 variieren zu können.

Durch die Vorrichtung 1 mit der Beleuchtungsvorrichtung 50 wird eine Technologieplattform zur Verfügung gestellt, mit der durch entsprechende Bestückung mit Platinen 56, 56' und Leuchtquellen 55 unterschiedliche Anwendungsfälle abgedeckt werden können, und mit der ein biologisches Medium im Bioreaktor mit Licht bestrahlt werden kann, insbesondere auch mit UV-Licht, IR-Licht, Laserlicht, moduliertem Licht usw. Eine Steuerung bzw. elektrische Leitung zur Stromversorgung der Lichtquellen 55 kann über die Rühröffnung und/oder die Kabelführung 13 ins Innere des Aufnahmebehälters 10 verlegt sein. Die obere und/oder untere Beleuchtungseinheit 51 bzw. 52 kann als im Wesentlichen flaches Profil, insbesondere Aluprofil, an die Innenseite der Außenbehälterwand 16 geklebt und/oder geschraubt sein.

Die Temperiereinheit 20 kann insbesondere dazu vorgesehen sein, die Aufnahmebehälterwand 16 auf eine Temperatur zwischen 10 und 60°, insbesondere von 27°C bis 37°C, zu temperieren. Der obere Teil des in Figur 4 gezeigten Aufnahmebehälters 10, in dem die obere Beleuchtungseinheit 51 angeordnet ist, kann an Stelle der Einblatttür 30 zwei Türen aufweisen, über die der Aufnahmebehälter 10 zumindest im oberen Teil vollständig öffenbar ist. Im Inneren jeder der beiden Türen ist ein halbringförmiges Element der oberen Beleuchtungseinheit 51 angeordnet.

Steuer- und/oder Stromkabel für die Beleuchtungsvorrichtung 50 kann entweder über die Rühröffnung oder durch eines der Fenster zu den Lichtquellen 55 verlegt sein.

Die Vorrichtung kann eine Sicherungsfunktion aufweisen, bei der automatisch die Beleuchtungsvorrichtung 50 ausgeschaltet wird, wenn die Tür(en) des Aufnahmebehälters 10 geöffnet wird.

In alternativen, in den Figuren nicht gezeigten Ausführungsformen der Beleuchtungsvorrichtung kann die Beleuchtungsvorrichtung zumindest ein flexibles LED-Band aufweisen, das entlang der Innenseite des Aufnahmebehälters angeordnet sein kann, z.B. entlang des Innenumfangs. Die LED-Bänder können im Wesentlichen horizontal entlang des Innenumfangs angeordnet sein und z.B. jeweils an der Türöffnung enden. Auch an der Innenseite der Einblatttür können LED-Bänder horizontal und/oder vertikal angeordnet sein, z.B. von einem Ende des Türblatts zu einem gegenüberliegenden Ende des Türblatts.

### Bezugszeichenliste

- 1: Vorrichtung
- 10: Aufnahmebehälter
- 12: Bodensichtfenster
- 13: Kabelführung
- 14: Rührvorrichtung
- 15: Auffangwanne
- 16: Aufnahmebehälterwand
- 18: Rollen
- 19: Fixierfüße
- 20: Temperierhohlwand
- 30: Einblatttür
- 32: Türsichtfenster
- 33: Türverdunklung
- 34: Türscharnier
- 35: Türgriff
- 36: Türöffnung
- 40: Füllstandhöhe
- 42: biologisches Medium
- 44: Einwegbehälter bzw. Einwegbeutel
- 50: Beleuchtungsvorrichtung
- 51: obere Beleuchtungseinheit
- 52: untere Beleuchtungseinheit
- 53: oberer Beleuchtungsträger
- 53a: Außenseite des oberen Beleuchtungsträgers
- 54: unterer Beleuchtungsträger
- 54a: Außenseite des unteren Beleuchtungsträgers
- 55: Lichtquelle
- 56: Platine
- 56': Platine
- 57: Steg
- bₕ: horizontale Ausdehnung eines Beleuchtungsträgers
- dₕ: horizontaler Innendurchmesser des Aufnahmebehälters

## Patentansprüche

1. Vorrichtung (1) zur Aufnahme eines Einwegbehälters (44) mit
- einem Aufnahmebehälter (10) mit zumindest einer Aufnahmebehälterwand (16), die einen Behälterinnenraum des Aufnahmebehälters (10) zum Aufnehmen des Einwegbehälters (44) begrenzt, und
- einer Temperiereinheit zum Temperieren der Aufnahmebehälterwand (16);
wobei an einer dem Behälterinnenraum zugewandten Innenseite der von der Temperiereinheit temperierten Aufnahmebehälterwand (16) eine Beleuchtungsvorrichtung (50) angeordnet ist, die dazu ausgebildet ist, Licht in den Behälterinnenraum des Aufnahmebehälters (10) abzustrahlen.

2. Vorrichtung nach Anspruch 1, wobei die Beleuchtungsvorrichtung (50) zumindest eine Lichtquelle (55) aufweist, die an einer Position der Innenseite der Aufnahmebehälterwand (16) angeordnet ist, die unmittelbar an einer dem Behälterinnenraum abgewandten Außenseite der Aufnahmebehälterwand (16) von der Temperiereinheit temperiert wird.

3. Vorrichtung nach Anspruch 1 oder 2, wobei die Beleuchtungsvorrichtung (50) eine Mehrzahl von Lichtquellen (55) aufweist, die über zumindest 50% der Fläche der Innenseiten aller lateralen Aufnahmebehälterwände (16) verteilt angeordnet sind; und/oder wobei die Beleuchtungsvorrichtung (50) eine Mehrzahl ansteuerbarer LEDs als Lichtquellen (55) aufweist.

4. Vorrichtung nach einem der vorangegangenen Ansprüche, wobei die Beleuchtungsvorrichtung (50) zumindest 90% der abgestrahlten Lichtleistung mit einer vorbestimmten Wellenlänge und/oder in einem vorbestimmten Wellenlängespektrum zwischen 50 nm und 50 µm abstrahlt.

5. Vorrichtung nach einem der vorangegangenen Ansprüche, wobei die Beleuchtungsvorrichtung (50) dazu ausgebildet ist, Licht mittels auswechselbaren Lichtquellen (55), die unterschiedliche Abstrahlspektren aufweisen, abzustrahlen.

6. Vorrichtung nach einem der vorangegangenen Ansprüche, wobei eine Außenform einer dem Behälterinnenraum abgewandten Außenseite (53a, 54a) der Beleuchtungsvorrichtung (50) an eine Innenform der Innenseite der Aufnahmebehälterwand (16) angepasst ist.

7. Vorrichtung nach Anspruch 6, wobei die Außenform der Beleuchtungsvorrichtung (50) konvex ausgebildet ist und die Innenform der Aufnahmebehälterwand (16) konkav ausgebildet ist.

8. Vorrichtung nach einem der vorangegangenen Ansprüche, wobei die Beleuchtungsvorrichtung (50) Licht mit einer Lichtleistung von bis zu etwa 3000 µmol/m²s in den Behälterinnenraum abstrahlt.

9. Vorrichtung nach einem der vorangegangenen Ansprüche, wobei die Beleuchtungsvorrichtung (50) eine Mehrzahl auf einem Streifen (53, 54) angeordneter Lichtquellen (55) aufweist, und wobei der Streifen (53; 54) im Wesentlichen vertikal entlang der Aufnahmebehälterwand (16) angeordnet ist.

10. Vorrichtung nach einem der vorangegangenen Ansprüche, wobei in einer Betriebsposition der Vorrichtung (1) der Einwegbehälter (44) in Berührkontakt mit der Beleuchtungsvorrichtung (50) steht;
und/oder
wobei über Lichtquellen (55) der Beleuchtungsvorrichtung (50) eine transparente Harzschicht und/oder Lackschicht mit einer Dicke von bis zu etwa 1 mm angeordnet ist.

11. Vorrichtung nach einem der vorangegangenen Ansprüche, wobei die Beleuchtungsvorrichtung (50) zumindest eine Wärmebrücke zwischen Lichtquellen (55) der Beleuchtungsvorrichtung (50) und der temperierten Aufnahmebehälterwand (16) aufweist.

12. Vorrichtung nach einem der vorangegangenen Ansprüche, wobei die Temperiereinheit eine Wärmeabfuhrleistung von bis zu mindestens 8 kW erbringt.

13. Vorrichtung nach einem der vorangegangenen Ansprüche, wobei die Aufnahmebehälterwand (16) zumindest teilweise als eine Temperierhohlwand (20) ausgebildet ist und die Temperiereinheit dazu ausgebildet ist, mit einem in der Temperierhohlwand (20) angeordneten Temperiermedium den Behälterinnenraum zu temperieren.

14. Vorrichtung nach einem der vorangegangenen Ansprüche, wobei der Aufnahmebehälter (10) dazu ausgebildet ist, Einwegbehälter (44) mit einem Volumen von zumindest etwa 500 Litern aufzunehmen.

15. Verfahren zur Aufnahme eines Einwegbehälters (44) mit den Schritten:
- Aufnehmen eines Einwegbehälters (44) in einen Behälterinnenraum eines Aufnahmebehälters (10) mit zumindest einer Aufnahmebehälterwand (16), die den Behälterinnenraum begrenzt;
- Beleuchten des im Behälterinnenraum angeordneten Einwegbehälters (44) mittels einer Beleuchtungsvorrichtung (50), die an einer dem Behälterinnenraum zugewandten Innenseite der Aufnahmebehälterwand (16) angeordnet ist; und
- Temperieren der Aufnahmebehälterwand (16) mittels einer Temperiereinheit.

## Claims

1. A device (1) for accommodating a disposable container (44) having
- a receptacle (10) with at least one receptacle wall (16), which limits a receptacle interior of the receptacle (10) for accommodating the disposable container (44), and
- a tempering unit for tempering the receptacle wall (16); wherein an illumination device (50) is arranged at an inside of the receptacle wall (16) tempered by the tempering unit facing the receptacle interior, which is designed to emit light into the receptacle interior of the receptacle (10).

2. The device according to claim 1, wherein the illumination device (50) has at least one light source (55), which is arranged at a position of the inside of the receptacle wall (16) which is directly tempered by the tempering unit at an outside of the receptacle wall (16) that is averted from the receptacle interior.

3. The device according to claim 1 or 2, wherein the illumination device (50) has a plurality of light sources (55), which are arranged distributed over at least 50% of the area of the insides of all lateral receptacle walls (16); and/or
wherein the illumination device (50) has a plurality of actuatable LEDs as light sources (55).

4. The device according to any of the foregoing claims, wherein the illumination device (50) emits at least 90% of the emitted light power with a predetermined wavelength and/or on a predetermined wavelength spectrum between 50 nm and 50 µm.

5. The device according to any of the foregoing claims, wherein the illumination device (50) is designed to emit light by means of replaceable light sources (55), which have different emission spectra.

6. The device according to any of the foregoing claims, wherein an external shape of an outside (53a, 54a) of the illumination device (50) averted from the receptacle interior is adapted to an internal shape of the inside of the receptacle wall (16).

7. The device according to claim 6, wherein the external shape of the illumination device (50) is convex and the internal shape of the receptacle wall (16) is concave.

8. The device according to any of the foregoing claims, wherein the illumination device (50) emits light with a light power of up to approximately 3000 µmol/m²s to the receptacle interior.

9. The device according to any of the foregoing claims, wherein the illumination device (50) has a plurality of light sources (55) arranged on a strip (53, 54), and wherein the strip (53, 54) is arranged substantially vertically along the receptacle wall (16).

10. The device according to any of the foregoing claims, wherein, in an operating position of the device (1), the disposable container (44) is in physical contact with the illumination device (50);
and/or
wherein a transparent resin layer and/or lacquer layer with a thickness of up approximately 1 mm is arranged above the light sources (55) of the illumination device (50).

11. The device according to any of the foregoing claims, wherein the illumination device (50) has at least one thermal bridge between light sources (55) of the illumination device (50) and the tempered receptacle wall (16).

12. The device according to any of the foregoing claims, wherein the tempering unit produces a heat dissipation capacity of up to at least 8 kW.

13. The device according to any of the foregoing claims, wherein the receptacle wall (16) is designed at least partially as a temperature control hollow wall (20) and the tempering unit is designed to temper the receptacle interior with a temperature-control medium arranged in the temperature control hollow wall (20).

14. The device according to any of the foregoing claims, wherein the receptacle (10) is designed to accommodate disposable containers (44) with a volume of at least approximately 500 liters.

15. A method for accommodating a disposable container (44) having the steps:
- accommodating a disposable container (44) in a receptacle interior of a receptacle (10) having at least one receptacle wall (16), which limits the receptacle interior;
- illuminating the disposable container (44) arranged in the receptacle interior by means of an illumination device (50), which is arranged on an inside of the receptacle wall (16) facing the receptacle interior; and
- tempering the receptacle wall (16) by means of a tempering unit.

## Revendications

1. Dispositif (1) de réception d'un récipient jetable (44) avec
- un récipient de réception (10) avec au moins une paroi de récipient de réception (16) qui délimite un espace intérieur de récipient du récipient de réception (10) pour recevoir le récipient jetable (44), et
- une unité de régulation de température pour réguler la température de la paroi de récipient de réception (16) ;
dans lequel un dispositif d'éclairage (50) est agencé contre un côté intérieur, tourné vers l'espace intérieur de récipient, de la paroi de récipient de réception (16) régulée par l'unité de régulation de température, lequel dispositif d'éclairage est conçu pour irradier de la lumière jusque dans l'espace intérieur de récipient du récipient de réception (10).

2. Dispositif selon la revendication 1, dans lequel le dispositif d'éclairage (50) présente au moins une source lumineuse (55) qui est agencée contre une position du côté intérieur de la paroi de récipient de réception (16) qui est directement régulée sur un côté extérieur de la paroi de récipient de réception (16) opposé à l'espace intérieur de récipient par l'unité de régulation de température.

3. Dispositif selon la revendication 1 ou 2, dans lequel le dispositif d'éclairage (50) présente une pluralité de sources lumineuses (55) qui sont agencées par répartition par-dessus au moins 50 % de la surface des côtés intérieurs de toutes les parois de récipient de réception (16) latérales ;
et/ou
dans lequel le dispositif d'éclairage (50) présente une pluralité de DEL qui peuvent être commandées en tant que sources lumineuses (55).

4. Dispositif selon l'une des revendications précédentes, dans lequel le dispositif d'éclairage (50) irradie au moins 90 % de la puissance lumineuse irradiée avec une longueur d'onde prédéterminée et/ou dans un spectre de longueurs d'onde prédéterminé entre 50 nm et 50 µm.

5. Dispositif selon l'une des revendications précédentes, dans lequel le dispositif d'éclairage (50) est conçu pour irradier la lumière au moyen de sources lumineuses (55) interchangeables qui présentent des spectres d'irradiation divers.

6. Dispositif selon l'une des revendications précédentes, dans lequel une forme extérieure d'un côté extérieur (53a, 54a), opposé à l'espace intérieur de récipient, du dispositif d'éclairage (50) est adaptée à une forme intérieure du côté extérieur de la paroi de récipient de réception (16).

7. Dispositif selon la revendication 6, dans lequel la forme extérieure du dispositif d'éclairage (50) est conçue de façon convexe et la forme intérieure de la paroi de récipient de réception (16) est conçue de façon concave.

8. Dispositif selon l'une des revendications précédentes, dans lequel le dispositif d'éclairage (50) irradie de la lumière jusque dans l'espace intérieur de récipient avec une puissance lumineuse allant jusqu'à 3 000 µmol/m²s environ.

9. Dispositif selon l'une des revendications précédentes, dans lequel le dispositif d'éclairage (50) présente une pluralité de sources lumineuses (55) agencées sur une bande (53, 54), et dans lequel la bande (53, 54), est agencée essentiellement à la verticale le long de la paroi de récipient de réception (16).

10. Dispositif selon l'une des revendications précédentes, dans lequel, dans une position de fonctionnement du dispositif (1), le récipient jetable (44) se trouve en contact d'affleurement avec le dispositif d'éclairage (50) ;
et/ou
dans lequel une couche de résine et/ou une couche de vernis transparente avec une épaisseur allant jusqu'à 1 mm est agencée par-dessus des sources lumineuses (55) du dispositif d'éclairage (50).

11. Dispositif selon l'une des revendications précédentes, dans lequel le dispositif d'éclairage (50) présente au moins un pont thermique entre des sources lumineuses (55) du dispositif d'éclairage (50) et la paroi de récipient de réception (16) dont la température est régulée.

12. Dispositif selon l'une des revendications précédentes, dans lequel l'unité de régulation de température apporte une puissance de dissipation thermique allant jusqu'à au moins 8 kW.

13. Dispositif selon l'une des revendications précédentes, dans lequel la paroi de récipient de réception (16) est conçue au moins partiellement en tant que paroi creuse de régulation de température (20) et l'unité de régulation de température est conçue pour réguler l'espace intérieur de récipient avec un milieu de régulation de température agencé dans la paroi creuse de régulation de température (20).

14. Dispositif selon l'une des revendications précédentes, dans lequel le récipient de réception (10) est conçu pour recevoir un récipient jetable (44) avec un volume d'au moins 500 litres environ.

15. Procédé de réception d'un récipient jetable (44) avec les étapes de :
- réception d'un récipient jetable (44) dans un espace intérieur de récipient d'un récipient de réception (10) avec au moins une paroi de récipient de réception (16) qui délimite l'espace intérieur de récipient ;
- éclairage du récipient jetable (44) agencé dans l'espace intérieur de récipient au moyen d'un dispositif d'éclairage (50) qui est agencé contre un côté intérieur, tourné vers l'espace intérieur de récipient, de la paroi de récipient de réception (16) ; et
- régulation de température de la paroi de récipient de réception (16) au moyen de l'unité de régulation de température.
